(19) Europäisches Patentamt
European Patent Office
Office européen des brevets

(11) Veröffentlichungsnummer: **0 002 026**
**B1**

(12) # EUROPÄISCHE PATENTSCHRIFT

(45) Veröffentlichungstag der Patentschrift: **04.11.81**

(51) Int. Cl.³: **C 07 C 102/08,** C 07 C 103/133

(21) Anmeldenummer: **78101326.3**

(22) Anmeldetag: **08.11.78**

(54) **Verfahren zur Herstellung von Carbonsäureamiden.**

(30) Priorität: **17.11.77 DE 2751336**

(43) Veröffentlichungstag der Anmeldung: **30.05.79 Patentblatt 79/11**

(45) Bekanntmachung des Hinweises auf die Patenterteilung: **04.11.81 Patentblatt 81/44**

(84) Benannte Vertragsstaaten: **BE CH DE FR GB NL**

(56) Entgegenhaltungen:
**DE-A-2 320 060**
**DE-A-2 611 061**
**DE-B-1 252 184**

(73) Patentinhaber: **BASF Aktiengesellschaft, Carl-Bosch-Strasse 38, D-6700 Ludwigshafen (DE)**

(72) Erfinder: **Platz, Rolf, Dr., Hansastrasse 5, D-6800 Mannheim 1 (DE)**
Erfinder: **Dockner, Toni, Dr., Grossgasse 6, D-6701 Meckenheim (DE)**
Erfinder: **Heners, Juergen, Dr., Waldstrasse 123, D-4450 Lingen (DE)**
Erfinder: **Herbert, Krug, Mussbacher Strasse 49, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 002 026 B1

BUNDESDRUCKEREI BERLIN

Verfahren zur Herstellung von Carbonsäureamiden

Die Erfindung betrifft ein Verfahren zur Herstellung von Carbonsäureamiden durch Wasseranlagerung an Nitrile in Gegenwart eines Kupfer- und Magnesiumsilikat enthaltenden und mit einem reduzierenden Gas behandelten Katalysators, dem noch Zement bestimmter Korngröße zugemischt wird.

Aus der US-PS 3 381 034 ist bekannt, daß man in Anwesenheit von Kupfer(II)- und Kupfer(I)-Salzen aus Nitrilen durch Wasseranlagerung Carbonsäureamide herstellen kann. Es wird beschrieben, daß man gegebenenfalls elementares Kupfer mitverwendet. Die Ausbeute an Endstoff ist unbefriedigend.

Die deutsche Offenlegungsschrift 2 001 903 lehrt, daß als Katalysator ein reduziertes Kupferoxid, reduziertes Kupfer-Chromoxid oder reduziertes Kupfer-Molybdänoxid bzw. entsprechende Mischungen für diese Reaktion verwendet werden. Die in den Beispielen beschriebenen Katalysatoren haben bei der technisch bedeutungsvollen Wasseranlagerung an Acrylnitril und Methacrylnitril eine unzureichende Selektivität. Beispiel 3 zeigt, daß zu Beginn der Reaktion die Ausbeute und die Umwandlung nur ungefähr 50% betragen; 30% des umgewandelten Nitrils erscheinen als β-Hydroxypropionitril. Erst nach längerer Reaktionszeit steigt die Umwandlung auf 90% und darüber. Auf der anderen Seite zeigt Beispiel 7, daß die Aktivität des reduzierten Oxids nach 1400 Stunden fällt, so daß dann nunmehr eine Ausbeute von 90% der Theorie erreicht wird. Auch die Anwesenheit von Stabilisierungsmitteln in Gestalt von Barium, wie Beispiel 5 zeigt, bringt keine befriedigenden Ergebnisse; man erhält einen Umsatz von 73%, der auf 43% fällt, und eine Ausbeute von 59%, die erst im Laufe der Reaktion auf 84% steigt. Man kann die zum Teil erheblichen Mengen an Nebenprodukten nicht bzw. nur umständlich abtrennen.

In der deutschen Offenlegungsschrift 2 036 126 ist ein Verfahren zur Herstellung eines Amids beschrieben, wobei an ein Nitril, z. B. Acrylnitril und Methacrylnitril, katalytisch Wasser angelagert wird. Genannt werden metallhaltige Katalysatoren wie Raney-Kupfer, Ullmann-Kupfer, reduzierte Kupferkatalysatoren, Kupfer mit einem Träger, Silber, Gold, Kobalt, Nickel, Palladium und Platin. Die in den Beispielen 1 bis 10 genannten Kupferkatalysatoren wie Raney-Kupfer, reduziertes Kupferoxid, Ullmann-Kupfer und Kupfer auf Asbestträger, liefern bei sehr niedrigen Umsätzen verdünnte Acrylamidlösungen. Die Dauer der Aktivität der Katalysatoren ist nicht angegeben. Im Falle der Verwendung des vergleichsweise teuren und umständlich herstellbaren Raney-Kupfers als Katalysator ist das Verfahren im Hinblick auf Wirtschaftlichkeit, einfache Betriebsweise und Handhabung des Katalysators unbefriedigend; Raney-Kupfer weist nur eine geringe Lebensdauer auf, und eine Regenerierung solcher Katalysatoren ist umständlich. Aus der deutschen Offenlegungsschrift 2 164 186 ist andererseits bekannt, daß alle diese Kupferkatalysatoren rasch entaktivieren und mit Wasserstoff bei erhöhter Temperatur regeneriert werden müssen.

Gegenstand der DE-OS 2 320 060 ist ein Verfahren zur Herstellung von Carbonsäureamiden durch Wasseranlagerung an Nitrile in Gegenwart eines kupferhaltigen Katalysators, wobei die Umsetzung mit einem Katalysator, der Kupfer und ein in Gegenwart einer Kupferverbindung durch Fällung einer Magnesiumverbindung mit einem Alkalisilikat hergestelltes Magnesiumsilikat enthält und mit einem reduzierenden Gas bei erhöhter Temperatur behandelt wird, durchgeführt wird.

Es wurde nun gefunden, daß sich das Verfahren der DE-OS 2 320 060 weiter ausgestalten läßt, wenn dem in vorgenannter Weise hergestellten und vom Fällungsmedium abgetrennten Magnesiumsilikat ein Zement von einer Korngröße von 1 bis 100 Mikrometern in einer Menge von 3 bis 50 Gewichtsprozent, bezogen auf Magnesiumsilikat, zugemischt wird.

Die Umsetzung wird für den Fall der Verwendung von Acrylnitril durch die folgenden Formeln wiedergeben:

$$CH_2{=}CH{-}CN + H_2O \longrightarrow CH_2{=}CH{-}\overset{\overset{\displaystyle O}{\|}}{C}{-}NH_2$$

Im Vergleich zu den bekannten Verfahren liefert das Verfahren nach der Erfindung auf einfacherem und wirtschaftlicherem Wege eine große Zahl von Carbonsäureamiden in guter Ausbeute und Reinheit und besserer Raum-Zeit-Ausbeute, bezogen auf die Gesamtkupfermenge im Katalysator, bei längerem Betrieb. Der Endstoff fällt meist in so reiner Form an, daß er sofort weiterverarbeitet werden kann. Umständliche Reinigungsoperationen werden vermieden. Die hohe Selektivität des Katalysators zusammen mit seiner Lebensdauer erlauben gerade im großtechnischen Maßstab längere Betriebszeiten und ersparen häufige Regenerierungen des Katalysators. Auch läßt sich der Katalysator einfacher und rascher regenerieren. Teure und schwer zugängliche Metalle werden nicht verwendet. Der erfindungsgemäße Katalysator ist kein Trägerkatalysator mit z. B. dem Träger Siliciumdioxid oder Magnesiumoxid; Magnesium und Magnesiumsilikat tragen in ihm zu den katalytischen Eigenschaften erheblich bei. Reduziert man auf einen Träger aufgebrachtes Kupferoxid mit Wasserstoff, so entsteht rotes Kupfer, reduziert man den erfindungsgemäßen Katalysator, so bildet sich schwarzes Kupfer. Der erfindungsgemäße Katalysator liefert wesentlich höhere Raum-Zeit-Ausbeuten und höhere

Selektivitäten, z. B. in den Ausbeuten an Acrylamid praktisch 100 Prozent. Eine solche quantitative Ausbeute wie bei dem erfindungsgemäßen Verfahren ist sehr wesentlich, da die Verunreinigungen von Acrylamid nicht oder nur schwer abzutrennen sind.

Im Vergleich zu dem in der deutschen Offenlegungsschrift 2 320 060 beschriebenen Verfahren kann das erfindungsgemäße Verfahren, gerade auch im großtechnischen Maßstab, mit wesentlich längerer Betriebsdauer durchgeführt werden. Der Katalysator ist vergleichsweise besser verformbar, gerade auch in Formlinge wie Tabletten, Pillen und Stränge, und bewahrt seine Form unter den Reaktionsbedingungen, gerade auch in Gegenwart von Wasser, während eines längeren Betriebs. Selbst nach Standzeiten von über 1000 bis 1500 Stunden in wäßriger Phase und gut durchströmten Reaktoren zerfallen z. B. tablettenförmige Katalysatoren nicht; ein ständiger Verlust an Katalysatorteilchen, die als feine Suspension ausgetragen werden, tritt so nicht ein. Verstopfungen des Reaktors mit diesen Partikeln, die mit zunehmender Betriebszeit den Druck im Reaktor ansteigen lassen und schließlich zum Katalysatorwechsel zwingen, werden vermieden. Alle diese vorteilhaften Eigenschaften des erfindungsgemäßen Verfahrens sind überraschend. Denn man hätte bei einer Zumischung von Zement, der ja bei Benetzung mit Wasser sich erhärtet, eine Verklebung, Verhärtung und Schrumpfung der porösen Katalysatormasse und damit während der Reaktion eine rasche Verringerung der katalytischen Oberfläche, insbesondere auch der inneren Oberfläche, Abnahme der Porengröße und Porenzahl und einen entsprechend raschen Ausbeuteverlust, eine verringerte Katalysatorselektivität, entsprechend häufigeren Katalysatorwechsel und verringerte Betriebszeiten erwarten müssen. Überraschend bleibt vergleichsweise die hohe Ausbeute des erfindungsgemäßen Verfahrens während der längeren Betriebsdauer praktisch konstant.

Als Ausgangsstoffe kommen aliphatische, cycloaliphatische, araliphatische und aromatische Mono-, Di- oder Polynitrile in Betracht. Bevorzugte Ausgangsstoffe sind solche der Formel

$$R(-CN)_x \qquad (I)$$

und dementsprechend bevorzugte Endstoffe sind solche der Formel

$$R\left(-\overset{\overset{\displaystyle O}{\|}}{C}-NH_2\right)_x \qquad (II)$$

worin x die Zahl 2 und insbesondere 1 und R einen Alkylrest mit 1 bis 6 Kohlenstoffen, einen Alkylenrest mit 1 bis 6, insbesondere 2 bis 6 Kohlenstoffatomen, einen Alkenylrest oder Alkenylenrest mit jeweils 2 bis 6 Kohlenstoffatomen, einen Cycloalkylrest oder Cycloalkylenrest mit 5 oder 6 Kohlenstoffatomen, einen Aralkylrest oder Aralkylenrest mit 7 bis 12 Kohlenstoffatomen, einen Phenylrest, Phenylenrest, Naphthylrest oder Naphthylenrest bezeichnet. Die vorgenannten Reste können noch durch unter den Reaktionsbedingungen inerte Gruppen, z. B. Alkylgruppen oder Alkoxygruppen mit jeweils 1 bis 4 Kohlenstoffatomen, substituiert sein.

Es kommen z. B. als Ausgangsstoffe in Betracht: Acetonitril, Propionitril, Cyclohexancarbonsäurenitril, Adipinsäuredinitril, Acrylnitril, Methacrylnitril, Crotonnitril, $\beta$-Phenylacrylnitril, Benzonitril, p-Toluylnitril, $\alpha$-Naphthoesäurenitril, Phthalsäuredinitril, Terephthalsäuredinitril, Isophthalsäuredinitril, Butyronitril, Maleinsäuredinitril, Glutardinitril, Bernsteinsäuredinitril, Valeriansäurenitril, Capronsäurenitril, Fumarsäuredinitril, $\beta$-Phenylessigsäurenitril, p-Äthoxy-benzontril.

Man kann den Ausgangsstoff mit Wasser in stöchiometrischer Menge oder im Überschuß, vorteilhaft in einem Verhältnis von 1 bis 50 Mol Wasser je Nitrilgruppe eines Mols des Ausgangsstoffes, umsetzen. Die Umsetzung wird in der Regel bei einer Temperatur von 50 bis 150, vorzugsweise 60 bis 110°C, drucklos oder unter Druck, kontinuierlich oder diskontinuierlich durchgeführt.

Der Katalysator enthält im wesentlichen Maße nicht ionisiertes Kupfer, das von vornherein, z. B. in Gestalt von frisch reduziertem Kupfer, dem Magnesiumsilikat vor der Behandlung mit reduzierenden Gasen (reduktive Behandlung) zugeführt wird oder zweckmäßiger sich erst aus Kupferverbindungen während der Behandlung bildet. Vorteilhaft wird Kupfer aus den Kupferverbindungen, die bei der Fällung des Magnesiumsilikats anwesend sind, stammen und durch die reduktive Behandlung in die nicht ionisierte Form übergehen. Im behandelten Katalysator können neben der Hauptmenge an nicht ionisiertem Kupfer, vorteilhaft von 0,001 bis 30, vorzugsweise von 0,01 bis 20, insbesondere von 0,1 bis 10 Gewichtsprozent des Gesamtkupfers, noch ein- und zweiwertiges Kupfer, z. B. in Gestalt der ursprünglichen Kupferverbindung oder einer bei der Behandlung gebildeten, wie Kupfersilikat, vorliegen. Im allgemeinen kommen Mengen von 50 bis 100, insbesondere von 70 bis 80 Gewichtsprozent Gesamtkupfer, bezogen auf die Gewichtsmenge im Katalysator enthaltenem Magnesiumsilikat, und 0,5 bis 2, insbesondere 1 bis 1,5 Mol Ausgangsnitril, bezogen auf das Grammatom im Katalysator enthaltenem Magnesium, in Frage.

Als Kupferverbindungen sind beispielsweise das Nitrat, Sulfat, Chlorid, Oxid, Hydroxid, Tartrat, Acetat, Oxalat, Cyanid, Cuprite wie Natriumcuprit, das Bromid, Jodid, Nitrit oder Carbonat des einwertigen oder zweckmäßig des zweiwertigen Kupfers geeignet.

Das Magnesiumsilikat wird in Gegenwart einer, zweckmäßig der das nicht ionisierte Kupfer liefernden Kupferverbindung durch Fällung einer Magnesiumverbindung mit einem Alkalisilikat, zweckmäßig einem Kaliumsilikat oder Natriumsilikat, in der Regel in einem wäßrigen Fällungsmedium, vorteilhaft in einem Verhältnis von 1 bis 5 Mol Alkalisilikat je Mol Magnesiumverbindung, hergestellt. Als Magnesiumverbindungen kommen in Frage: Magnesiumnitrat, -sulfat, -chlorid, -tartrat, -acetat, -oxalat, -bromid, -jodid, -nitrit. Die Fällung wird vorteilhaft bei 15 bis 50°C durchgeführt. Die Kupferverbindung kann im Fällungsmedium teilweise suspendiert sein.

Der Katalysator kann kleine Mengen von Zink, Cadmium, Chrom, Molybdän, Wolfram, Vandin, Uran, Titan und/oder Thorium in Gestalt der Metalle oder zweckmäßig entsprechender Verbindungen enthalten. Vorteilhaft kommen Mengen von 1 bis 30, insbesondere von 1 bis 10 Gewichtsprozent Zusatzmetall, bezogen auf die Gewichtsmenge im Katalysator enthaltenem Magnesium, in Frage. Als Verbindungen werden zweckmäßig die Nitrate, Sulfate, Chloride, Tartrate, Acetate, Oxalate, Bromide, Jodide, Nitrite vorgenannter Metalle verwendet. In der Regel werden diese Verbindungen schon im Fällungsmedium der Magnesiumverbindung anwesend sein.

In der bevorzugten Ausführungsform werden Katalysatoren, die durch Fällung einer Magnesiumverbindung in Gegenwart der Kupferverbindung und gegebenenfalls einer oder mehrerer Verbindungen der Zusatzmetalle in wäßrigem Medium nach dem in dem deutschen Reichspatent 869 052 beschriebenen Verfahren der Katalysatorherstellung erhalten werden, verwendet. In manchen Fällen ist es zweckmäßig, die gefällte Masse nach dem Waschen und Trocknen noch mit einer nicht zu großen Menge Alkalisilikatlösung, zweckmäßig 5 bis 20 Gewichtsprozent einer 10- bis 30gewichtsprozentigen Natrium- oder Kaliumsilikatlösung, bezogen auf gefällte Masse, durchzukneten und nach dem abermaligen Trocknen bei möglichst nicht zu hohen Temperaturen, z. B. bei 15 bis 30°C zu verformen. Ein Zusatz von zu großen Mengen Alkalisilikat kann unter Umständen zu unerwünschten Nebenreaktionen führen, weshalb es oftmals zweckmäßig ist, erst nach dem Verformen die Katalysatoren nur kurz mit verdünnter Wasserglaslösung bzw. vorgenannten Lösungen zu behandeln.

Vorteilhaft werden 10- bis 50gewichtsprozentige, wäßrige Lösungen genannter Kupfer-, Magnesium- und gegebenenfalls Zusatzmetallverbindungen und 10- bis 30gewichtsprozentige, wäßrige Lösungen von Alkalisilikat miteinander bei vorgenannter Fällungstemperatur gemischt und wird während 1 bis 60 Minuten die Fällung durchgeführt. Dann wird der Niederschlag zweckmäßig abgesaugt, mit Wasser gewaschen, z. B. bis zur Entfernung des ursprünglichen Kupferanions wie des Nitratanions. Man kann dann den Niederschlag bei 20 bis 30°C vortrocknen, verformen, z. B. in Tabletten, Kugeln, Strangpreßlinge, und dann bei 50 bis 70°C trocknen. Im allgemeinen wird man aber vorteilhafter den abgesaugten und gegebenenfalls gewaschenen Niederschlag trocknen, ihm den Zement zumischen und das Gemisch verformen. Zweckmäßig erfolgt anschließend die reduzierende Behandlung.

Der so erhaltene Katalysator wird bei erhöhter Temperatur, zweckmäßig bei 100 bis 230°C, vorzugsweise bei 180 bis 230°C, drucklos oder unter Druck, kontinuierlich oder diskontinuierlich mit reduzierenden Gasen behandelt. In der Regel wird man als reduzierendes Gas Wasserstoff verwenden. Es kommen aber auch andere reduzierende Gase wie Kohlenmonoxid, Olefine, z. B. Äthylen, Propylen, oder Gasgemische oder gereinigte oder ungereinigte gasförmige Gemische (technisches Gas), die reduzierende Gase wie Wasserstoff oder Kohlenmonoxid enthalten, in Frage, z. B. Brenngase wie Leuchtgas, Generatorgase, Kohlenschwelgase, Kohlengas, Gichtgas, Schwelgeneratorgase, Wassergase, Ölgas, Kokereigas (Ferngas), Stadtgase, Synthesegase, technisches Propan oder technisches Butan. Die Behandlungszeit beträgt zweckmäßig 0,5 bis 24, vorzugsweise 1 bis 15 Stunden. Vorteilhaft wird der Katalysator nach Herstellung und Trocknung zuerst unter Stickstoff, vorzugsweise in einem Stickstoffstrom, bei erhöhter Temperatur, vorzugsweise bei 100 bis 180°C, insbesondere 120 bis 140°C, gehalten, zweckmäßig während 0,5 bis 3 Stunden. Dann erfolgt die reduktive Behandlung, vorzugsweise während 0,5 bis 24 Stunden mit 5 bis 15 Mol Wasserstoff oder entsprechendem Äquivalent reduzierendem Gas je Kilogramm Katalysator. In einer bevorzugten Ausführungsform wird in den Stickstoffstrom der Vorbehandlung unter gleichzeitiger Erhöhung der Temperatur auf die Behandlungstemperatur wachsende Mengen Wasserstoff eingeführt und die Stickstoffeinleitung entsprechend verringert, so daß z. B. nach 6 bis 20 Stunden anstelle des reinen Stickstoffstroms ein Gasstrom, der nur noch das reduzierende Gas bzw. an Stickstoff nur die üblicherweise in diesem reduzierenden Gas enthaltene Menge enthält, verwendet wird. Nach der Reduktion enthält der Katalysator vorteilhaft nur noch 0 bis 1, vorzugsweise von 0 bis 0,1 Gewichtsprozent Kupfer in Gestalt von Kupferoxid, bezogen auf Gesamtkupfer, 0 bis 49, vorzugsweise von 0,1 bis 30 Gewichtsprozent Kupfer in Gestalt von Kupfersilikat und 51 bis 100, vorzugsweise von 70 bis 100 Gewichtsprozent Kupfer in Gestalt von nicht ionisiertem Kupfer, bezogen auf Gesamtkupfer.

Die Zumischung des Zements und/oder die Verformung, z. B. die Herstellung von Körnern, Würfeln, Zylindern, Ringen, Sternen und insbesondere von Tabletten, Kugeln, Pillen, Strangpreßlingen aus der Katalysatormasse, können an mehreren oder in der Regel an einer Stelle innerhalb der Reihenfolge der Operationen zur Katalysatorherstellung erfolgen. Zweckmäßig ist die folgende Reihenfolge: a) Fällung, b) Absaugen oder Filtration des Niederschlags, c) gegebenenfalls Wäsche, in der Regel mit Wasser, zweckmäßig bei einer Temperatur von 20 bis 50°C, d) Trocknen bei 10 bis 100°C, vorzugsweise

bei 15 bis 75° C.

Zwar kann die Verformung vor der Zumischung des Zements erfolgen und auch beide Operationen voneinander durch eine oder mehrere Operationen getrennt sein, in der Regel wird man aber zweckmäßig die Verformung anschließend an die Zementzumischung durchführen. Die Zumischung — und damit die anschließende Verformung — legt man zweckmäßig nach dem Trocknen des Niederschlags.

Bei der Zumischung werden Zement und zweckmäßig Mischzusätze verwendet. Unter Zement werden hier Stoffe im Sinne der Definition von Zementen nach Ullmanns Encyklopädie der technischen Chemie, Band 19, Seiten 1 bis 31, verstanden. Geeignete Zemente sind z. B. Portlandzement, Eisenportlandzement, Hochofenzement, Traßzement, Sulfathüttenzement, Ferrarizement, Erzzement, Kühlzement, Suevit-Traß-Zement, Traßhochofenzement, Ölschieferzement, Tiefbohrzement, Quellzement, Tonerdezement. Die Zusammensetzung solcher Zemente ist zweckmäßig 20 bis 69 Gewichtsprozent CaO, 0,2 bis 30 Gewichtsprozent $SiO_2$, 5 bis 75 Gewichtsprozent $Al_2O_3+TiO_2$, 0 bis 8 Gewichtsprozent $Fe_2O_3$ bzw. FeO, 0 bis 2 Gewichtsprozent $Mn_2O_3$ (MnO), 0 bis 3 Gewichtsprozent Alkalioxide, 0,2 bis 7 Gewichtsprozent MgO, 0 bis 9 Gewichtsprozent $SO_3$. Bezüglich der Herstellung und Eigenschaften der vorgenannten Zemente wird auf Ullmanns Werk (loc. cit.) verwiesen.

Bevorzugt hat die Katalysatormasse (Magnesiumsilikat) vor dem Vermischen eine spezifische Oberfläche von 100 bis 200, vorzugsweise von 120 bis 170 $m^2/g$, Porenvolumen von 0,3 bis 1,0, vorzugsweise von 0,6 bis 0,9 $cm^3/g$, einen durchschnittlichen Porenradius von 200 bis 800, vorzugsweise von 400 bis 620 Nanometern. Der Zement hat vor dem Vermischen bevorzugt eine spezifische Oberfläche von 0,5 bis 5, vorzugsweise von 0,8 bis 2 $m^2/g$, eine Schüttdichte von 800 bis 900 g/l, eine Korngröße von 2 bis 70, vorzugsweise von 2,5 bis 50, insbesondere von 3 bis 30 Mikrometern, der fertige, geformte Katalysator nach der Reduktion bevorzugt eine spezifische Oberfläche von 80 bis 180, vorzugsweise von 100 bis 170 $m^2/g$, ein Porenvolumen von 0,19 bis 0,3, vorzugsweise von 0,22 bis 0,28 $cm^3/g$, eine Korngröße von 100 bis 400, vorzugsweise von 100 bis 300 Mikrometern. Gegebenenfalls können Katalysatormasse oder Zement noch z. B. durch Mahlen auf die vorgenannten Werte zerkleinert werden.

Als Mischzusatz wird im allgemeinen Wasser (Anmachwasser), vorzugsweise mit einer Wassermenge im Gewichtsverhältnis Wasser zu Zement (Wasserzementwert) von 0,4 bis 1,5, insbesondere von 0,8 bis 1,3 zu 1 verwendet. Zweckmäßig kommen als weitere Mischzusätze Graphit, Talkum und/oder Fettsäuren wie Stearinsäure, zweckmäßig in einer Menge von 1 bis 10, vorzugsweise von 1 bis 5 Gewichtsprozent, bezogen auf Zement, in Betracht, um bei der Verformung als Schmiermittel zu wirken.

Der Zement wird in einer Menge von 3 bis 50, vorzugsweise von 5 bis 20 Gewichtsprozent, bezogen auf Magnesiumsilikat, zugemischt. Bei diesem Verhältnis wird Magnesiumsilikat als die der Gesamtmenge an bei der Fällung anwesendem Magnesium theoretisch entsprechenden Menge an $MgSiO_3$ definiert, gleichgültig, ob die tatsächlich erhaltene Menge an Mangesiumsilikat in der Struktur und in der Maximalmenge davon abweicht.

Die Zumischung wird im allgemeinen bei 10 bis 50, vorzugsweise 20 bis 40° C, drucklos oder unter Druck, kontinuierlich oder diskontinuierlich durchgeführt. Sie erfolgt z. B. in folgender Weise: Ein Gemisch von Zement, Wasser und gegebenenfalls weitere Mischungszusätze werden der Katalysatormasse nach der Trocknung zugegeben. Man kann dann die Verformung in mehreren, z. B. 2 Schritten, oder vorteilhaft in einem Schritt durchführen. Die Verformung wird zweckmäßig bei einer Temperatur von 10 bis 50, vorzugsweise beim einstufigen Verfahren von 15 bis 30° C durchgeführt. Der fertige Formling kann zweckmäßig erneut mit Wasser in Berührung kommen, z. B. durch Liegenlassen bei 15 bis 30° C an feuchter Luft oder Eintauchen in Wasser bei 15 bis 30° C. Die Formlinge werden zweckmäßig naß in Folienbeuteln verschlossen und dort 4 Tage lang bei Zimmertemperatur aufbewahrt. Dann trocknen sie zweckmäßig 7 Tage an der Luft bei 20° C. Zuletzt werden sie zweckmäßig 11 Tage lang bei 50° C getempert. Zweckmäßig erfolgt nun die reduktive Behandlung des Formlings in vorgenannter Weise.

Die Umsetzung kann wie folgt durchgeführt werden: Ausgangsnitril, Wasser und der in vorgenannter Weise hergestellte und reduktiv behandelte Katalysator werden während 1 bis 4 Stunden bei der Reaktionstemperatur gehalten. Dann wird der Endstoff aus dem Reaktionsgemisch in üblicher Weise, z. B. durch Filtration, Einengen des Filtrats und erneuter Filtration abgetrennt.

Die nach dem Verfahren der Erfindung erhältlichen Carbonsäureamide sind wertvolle Ausgangsstoffe für die Herstellung von Lösungsmitteln, Flockungsmitteln, Vernetzungsmitteln, Schädlingsbekämpfungsmitteln, Pflanzenschutzmitteln, Textilhilfsmitteln, z. B. Avivagemitteln, Hydrophobiermitteln, Schaumstabilisatoren, Waschmitteln. Amide höherer Fettsäuren sind Tenside und Avivagemitttel. Ungesättigte Carbonsäureamide, z. B. Acrylsäureamid und Methacrylsäureamid, sind bekannte Monomere für Kunststoffe. Bezüglich der Verwendung wird auf vorgenannte Veröffentlichungen und Ullmanns Encyklopädie der technischen Chemie, Band 14, Seiten 287 ff, und Ergänzungsband, Seite 136, verwiesen.

Die in den folgenden Beispielen aufgeführten Teile bedeuten Gewichtsteile. Sie verhalten sich zu den Volumteilen wie Kilogramm zu Liter.

## Beispiel 1

### a) Herstellung des Katalysators

Wäßrige, 20gewichtsprozentige Lösungen von 160 Teilen Kupfernitrat, 100 Teilen Magnesiumnitrat und 8 Teilen Chromnitrat werden miteinander gemischt und dann mit einer wäßrigen, 15gewichtsprozentigen Lösung von 220 Teilen Kaliumsilikat bei 20°C versetzt. Die gebildete Suspension wird abgesaugt und das Filtergut mit Wasser gewaschen, bis die Waschflüssigkeit keine Nitratanionen mehr enthält. Das Filtergut wird bei 40°C während 5 Stunden getrocknet. Das getrocknete Gut (90 Teile) wird gemahlen und mit 5 Teilen Zement und 15 Teilen Wasser bei 20°C gemischt. Das Filtergut hat vor dem Vermischen eine spezifische Oberfläche von 150 $m^2/g$, ein Porenvolumen von 0,90 $cm^3/g$, einen Porenradius von 610 Nanometern. Der Zement hat vor dem Vermischen eine spezifische Oberfläche von 1 $m^2/g$, eine Schüttdichte von 850 g/l. Anschließend wird das Pulver bei 20°C zu Tabletten 5 × 3 mm unter einem Druck von 800 bar verformt. Die Formlinge werden naß in Folienbeuteln verschlossen und 4 Tage lang bei 20°C aufbewahrt. Danach trocknen sie 7 Tage an der Luft. Die reduktive Behandlung wird mit dem Erhitzen der Tabletten im Stickstoffstrom während 60 Minuten bei 130°C und 1 bar eingeleitet. Dann gibt man dem Stickstoff Wasserstoff in steigendem Maße zu. Innerhalb 2 Stunden ist der gesamte Stickstoff durch Wasserstoff ersetzt, wobei die Temperatur auf 220°C ansteigt. Anschließend wird der Katalysator noch während 3 Stunden bei 220°C in dem reinen Wasserstoffstrom gehalten und abgekühlt. Der so erhaltene Katalysator(1000 Teile), der 300 Teile Gesamtkupfer und davon 299 Teile nicht ionisiertes Kupfer enthält, wird in den folgenden Beispielen verwendet; er hat eine spezifische Oberfläche von 135 $m^2/g$, ein Porenvolumen von 0,23 $cm^3/g$ und eine Stirnflächenbruchfestigkeit von 25 bar.

### b) Reaktion

In einem Rohrreaktor wird die katalytische Wasseranlagerung kontinuierlich durchgeführt. Der Reaktor hat 2 Meter Länge und 50 Millimeter Durchmesser und ist mit 1000 Teilen in vorgenannter Weise hergestellten Katalysator gefüllt. Pro Stunde werden dem Reaktor von unten nach oben 160 Teile sauerstofffreies Acrylnitril und 500 Teile sauerstofffreies Wasser über Dosierpumpen zugeführt. Die Reaktionstemperatur wird auf 70°C gehalten. Das flüssige Reaktionsgemisch leitet man über einen Abscheider, einen Kühler und einen weiteren Abscheider. Man erhält stündlich, nach Filtration und Einengen des Filtrats, 7,76 Teile Acrylnitril und 22,1 Teile Acrylamid vom Kp. 84,5°C. Dies entspricht einem Umsatz von 68 Prozent, die Ausbeute ist praktisch 100 Prozent.

## Beispiel 2

Man verfährt wie in Beispiel 2, unter Verwendung eines Katalysators mit 10 Teilen Zement. Der Zement hat eine spezifische Oberfläche von 2 $m^2/g$ und eine Schüttdichte von 850 g/l. Der Katalysator hat eine spezifische Oberfläche von 125 $m^2/g$, ein Porenvolumen von 0,24 $cm^3/g$ und eine Stirnflächenbruchfestigkeit von 65 kg/qcm Stirnfläche. Die eingesetzten Tabletten haben nach 4000 Stunden ihre ursprüngliche Form behalten. Man erhält stündlich, nach Filtration und Einengen des Filtrats, 9,48 Teile Acrylnitril und 19,8 Teile Acrylamid vom Kp. 84,5°C, entsprechend einem Umsatz von 61 Prozent und einer Ausbeute von > 99% der Theorie.

## Beispiel 3

Man verfährt wie in Beispiel 1, verwendet aber einen Katalysator mit 20 Teilen Zement. Der Zement hat eine spezifische Oberfläche von 2 $m^2/g$ und eine Schüttdichte von 800 bis 900 g/l. Der Katalysator hat eine spezifische Oberfläche von 100 $m^2/g$, ein Porenvolumen von 0,20 $cm^3/g$ und eine Stirnflächenbruchfestigkeit von 81 bar. Nach 1000 Betriebsstunden beträgt die Stirnflächenbruchfestigkeit 74 bar und fällt nach 4000 Betriebsstunden auf 68 bar zurück. Die Form des eingesetzten Katalysators bleibt erhalten. Man erhält stündlich, nach Filtration und Einengen des Filtrats, 11,43 Teile Acrylnitril und 17,2 Teile Acrylamid vom Kp. 84,5°C, entsprechend einem Umsatz von 53 Prozent und einer Ausbeute von > 99% der Theorie.

## Beispiel 4
(Vergleich)

Man verfährt wie in Beispiel 1, setzt aber einen zementfreien Katalysator mit einer Stirnflächenbruchfestigkeit von 20 bar ein. Der Katalysator hat eine spezifische Oberfläche von

150 $m^2/g$, ein Porenvolumen von 0,88 $cm^3/g$, einen Porenradius von 570 Nanometern. Nach 1000 Betriebsstunden beträgt die Stirnflächenbruchfestigkeit 16 bar und fällt nach 2000 Stunden auf 10 bar zurück. Nach 3000 Stunden ist der größte Teil der eingebrachten Tabletten zerfallen. Man erhält stündlich, nach Filtration und Einengen des Filtrats, 7,3 Teile Acrylnitril und 22,7 Teile Acrylamid vom Kp. 84,5° C, entsprechend einem Umsatz von 70 Prozent und einer Ausbeute von >99% der Theorie.

Tabelle

| Beispiel | Stirnflächenbruchfestigkeit (kg/qcm Stirnfläche) | | | | |
|---|---|---|---|---|---|
| | nach 1000 Stunden | 2000 Stunden | 3000 Stunden | 4000 Stunden | Ausbeute % der Theorie |
| 1 | 25 | 14 | 10% des Katalysators zerfallen | – | über 99 |
| 2 | 65 | 58 | 53 | 50 | über 99 |
| 3 | 81 | 74 | 71 | 68 | über 99 |
| 4 (Vergleich) | 20 | 10 | völlig zerfallen | – | über 99 |

Die Katalysatoren werden in Tablettenform auf der Stirnfläche mit zunehmendem Gewicht belastet, bis sie brechen. Das Gewicht pro belasteter Stirnfläche wird, in kg/qcm Stirnfläche (Stirnflächenbruchfestigkeit) ausgedrückt, gemessen. Als Prüfmaschine verwendet man die in DIN 51 223 angegebenen.

**Patentanspruch**

Verfahren zur Herstellung von Carbonsäureamiden durch Wasseranlagerung an Nitrile in Gegenwart eines kupferhaltigen Katalysators, wobei die Umsetzung mit einem Katalysator, der Kupfer und ein in Gegenwart einer Kupferverbindung durch Fällung einer Magnesiumverbindung mit einem Alkalisilikat hergestelltes Magnesiumsilikat enthält und mit einem reduzierenden Gas bei erhöhter Temperatur behandelt wird, durchgeführt wird, dadurch gekennzeichnet, daß dem in vorgenannter Weise hergestellten und vom Fällungsmedium abgetrennten Magnesiumsilikat ein Zement von einer Korngröße von 1 bis 100 Mikrometern in einer Menge von 3 bis 50 Gewichtsprozent, bezogen auf Magnesiumsilikat, zugemischt wird.

**Claim**

A process for the preparation of carboxamides by addition of water to nitriles in the presence of a copper-containing catalyst, the reaction being carried out using a catalyst which contains copper and a magnesium silicate prepared in the presence of a copper compound by precipitating a magnesium compound with an alkali metal silicate and which has been treated with a reducing gas at elevated temperature, characterised in that the magnesium silicate prepared in the way described above and separated from the precipitant is mixed with 3 to 50 percent by weight, based on magnesium silicate, of a cement having a particle size of 1 to 100 micrometers.

**Revendication**

Procédé de préparation d'amides d'acides carboxyliques par addition d'eau sur des nitriles en présence d'un catalyseur au cuivre, la réaction étant réalisée avec un catalyseur contenant du cuivre et un silicate de magnésium préparé par précipitation d'un dérivé du magnésium par un silicate de métal alcalin en présence d'un dérivé du cuivre et traité à température accrue par un gaz réducteur, caractérisé en ce que l'on mélange au silicate de magnésium préparé comme indiqué et séparé du milieu de précipitation entre 3 et 50% de son poids d'un ciment d'une granulométrie comprise entre 1 et 100 microns.